# EUROPEAN PATENT SPECIFICATION

(11) **EP 1 977 216 B1**
(45) Date of publication and mention of the grant of the patent: **27.12.2017**
(21) Application number: 07716549.6
(22) Date of filing: 10.01.2007
(51) Int. Cl.: G01N 19/00, G01N 3/32, A61F 2/46, A61F 2/44, G01N 3/56, A61F 2/76, F16D 3/72, F16D 3/76, G01M 99/00

(54) **TRANSMISSION FOR TORQUE TRANSFER WITH AXIAL COMPLIANCE**
ÜBERSETZUNG FÜR DREHMOMENTÜBERTRAGUNG MIT AXIALER NACHGIEBIGKEIT
TRANSMISSION POUR TRANSFERT DE COUPLE AVEC COMPLIANCE AXIALE

(30) Priority: 20.01.2006 US 335974; 13.01.2006 US 332407; 20.01.2006 US 760595 P
(43) Date of publication of application: 08.10.2008
(73) Proprietor: MTS SYSTEMS CORPORATION, Eden Prairie, MN 55344 (US)
(72) Inventor: SCHULZ, Bradley, D., Savage, MN 55378 (US); LESKA, Paul, J., Chanhassen, MN 55317 (US); WILLIS, Dennis, J., Bloomington, MN 55437 (US); FAHRENDORFF, Harold, F., Golden Valley, MN 55416 (US)
(74) Representative: Vossius & Partner Patentanwälte Rechtsanwälte mbB
(86) International application number: PCT/US2007/000726
(87) International publication number: WO 2007/084326

(56) References cited:
- GB-A- 1 024 245
- US-A- 3 658 143
- US-A- 4 196 635
- US-A1- 2005 056 099
- US-A1- 2005 241 404
- MICKLEY K ET AL: "AN EXPERIMENTAL TEST-RIG FOR THE BIOMECHANICAL ANALYSIS OF HUMAN SPINE SEGMENTS", REPORTS IN APPLIED MEASUREMENT, HOTTINGER BALDWIN MESSTECHNIK. DARMSTADT, DE, vol. 6, no. 2, 1 January 1990 (1990-01-01) , pages 43-49, XP000249812, ISSN: 0930-7923

## Description

### FIELD

The embodiments of the present invention relate to the field of transmissions for torque transfer, and to machines that employ such transmissions, such as orthopedic device wear test machines.

### BACKGROUND

There are many applications in which a transfer of torque is required. In many devices, it is desirable to transmit pure torque through a machine component with high torsional stiffness in a very controlled and repeatable manner.

For example, testing machines, such as orthopedic device wear testing machines, will often have a component to which torque is applied repeatedly. After a number of cycles in which torque is transmitted to the orthopedic device implant, numbering in the millions, for example, wearing of the orthopedic device implant test specimen is determined. It is critical in such testing machines that the torque be transmitted with little deflection related error and be repeatable. In an orthopedic device implant wear testing machine, for example, a plurality of test stations are provided in which test specimens are tested. A precise and consistently repeatable torque transmission must be applied to each of the test specimens over the millions of test cycles so that each of the test specimens are subjected to the same wear conditions. Otherwise, the validity of the testing will be in question.

GB 1 024 245 A discloses a machine for testing tubular specimens. The machine includes a hydraulic ram by which axial tension can be applied to the specimen, two opposed hydraulic rams acting on a drum whereby pure torque can be applied to the specimen, and a pair of pistons linked together by a tie and working in bores of the same diameter as a bore of the specimen and into which the latter bore opens to confine liquid under pressure admitted to the interior of the specimen without any axial load being imposed on the specimen from this source.

US 4 196 635 A discloses a test apparatus for the simultaneous loading of a test sample with longitudinal forces and with torque. A piston rod of a longitudinal loading cylinder and a piston of a torque cylinder are coupled for relative axial movement by a fork connected to the piston rod of the longitudinal loading cylinder and guided in a torque transmitting member connected to the piston of the torque cylinder.

K. Mickley et al., "An experimental test-rig for the biomechanical analysis of human spine segments", Reports in Applied Measurement, HOTTINGER BALDWIN MESSTECHNIK. DARMSTADT, DE, (19900101), vol. 6, no. 2, ISSN 0930-7923, pages 43 - 49, discloses a technique to analyze biomechanical parameters of human vertebrae using force and inductive displacement transducers.

In certain devices, such as in orthopedic device wear test machines, a torque transfer transmission to provide precise force and motion control and rigid torque transfer along with axial loading. One such orthopedic device wear testing machine is a spinal implant wear test machine. In such a machine, for example, axial loading is provided on the spinal implant under test, and a torque is also applied to the test specimen. In such devices, it is desirable to provide high torsional stiffness with little deflection related error, while at the same time providing low axial stiffness so that crosstalk is not seen at the load cell.

### SUMMARY

There is a need for a transmission for torque transfer that allows the transmission of pure torque to a machine component with high torsional stiffness and high axial compliance. There is also a need for an orthopedic device test machine that employs a torque transmission that is axially compliant and torsionally stiff to provide rigid torque transfer to a rotational element with axial compliance to allow substantially friction free axial translation of the rotational element.

A transmission for torque transfer as defined by the independent claim and and an orthopaedic device test machine comprising the transmission for torque transfer are provided. The dependent claims define embodiments.

### BRIEF DESCRIPTION OF THE DRAWINGS

Figure 1 is a schematic depiction of a torque transmitter depicting the transmission of the torque and axial compliance in accordance with embodiments of the present invention.
Figure 2 is a perspective view of an exemplary test machine constructed in accordance with embodiments of the present invention, employing a transmission for torque transfer in accordance with embodiments of the present invention.
Figure 3 schematically depicts the motion of vertical load actuators in the test machine of Figure 3.
Figure 4 schematically depicts axial rotation motion in accordance with embodiments of the present invention.
Figure 5 is a perspective view of a torque transfer transmission constructed in accordance with embodiments of the present invention, shown in isolation.
Figure 6 is a cross-sectional view of one embodiment of a transmission for torque transfer.
Figure 7 shows a rear view of the test machine of Figures 2-4.

### DETAILED DESCRIPTION

The embodiments of the present invention address and solve problems related to the providing of pure torque to a machine component with high torsional stiffness and high axial compliance, without inducing frictional forces. Such a torque transfer transmission is especially useful in testing machines, such as orthopedic device testing machines, including spinal implant wear testing machines. The embodiments of the invention provide precise force and motion control by employing a torque transmission that transmits pure torque to a machine component with high torsional stiffness and high axial compliance. A rotational element is configured for rotation with respect to a non-rotational element. This rotational element has an axis of rotation and is movable axially along the axis of rotation with respect to the non-rotational element. A rotational transfer link is provided that is rotatable about the axis of rotation and is drivable by an actuator. A flexible connector is coupled between the rotational transfer link and the rotational element. This flexible connector is flexible along the axis of rotation, but is torsionally stiff so as to rigidly transfer torque between the rotational transfer link and the rotational element. It does this without inducing friction to the relative axial movement between the rotational element and the non-rotational element. When the torque transfer transmission is coupled to a friction free axial actuator, as in certain embodiments, the transmission provides a friction free axial/torsion actuation system. The axial portion in the system is allowed to translate along its axis freely due to the high axial compliance of the transmission while the transmission simultaneously facilitates rigid torque transfer.

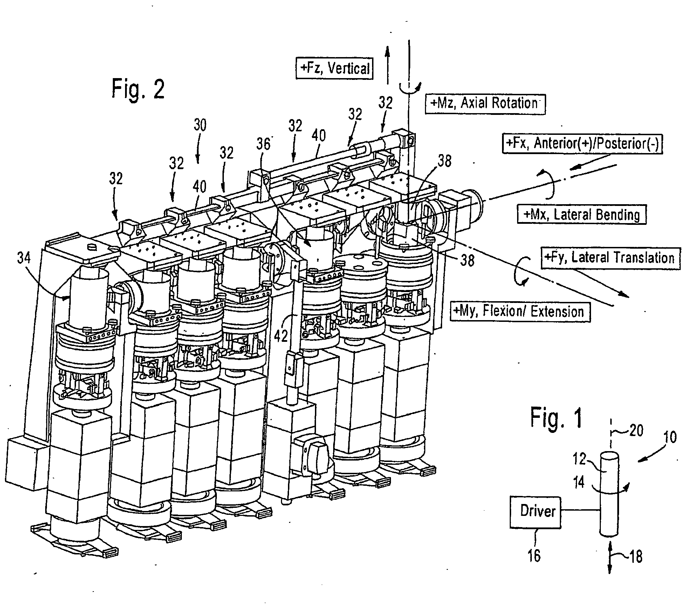

Figure 1 is a schematic depiction of a torque transfer transmission with the direction of motions shown for explanation purposes. In Figure 1, the torque transfer transmission 10 includes a rotational element 12 that rotates in the direction of arrow 14. The rotational element 12, such as a shaft, is driven by a driver 16. The arrows 18 schematically represent the axially compliant nature of the rotational element 12. In other words, the rotational element 12, in addition to its rotary motion 14 provided by driver 16, is able to freely translate along axis 20. As well, the rotational element 12 may also be controllably translated along axis 20, such as by a vertical force applicator (not shown). The axial compliance provided to the rotational element serves to prevent induction of frictional forces by the torque transmission. The axial compliance is important in order to limit the cross-talk onto the vertical load to a very small amount, and prevent cross-talk from being seen at a load cell, for example. At the same time, the transmission provided by the driver 16 to the rotational element 12 has high torsional stiffness, which is desirable to transmit drive torque with little deflection related error.

Figure 2 shows a front perspective view of a test machine constructed in accordance with embodiments of the present invention, which employ a torque transfer transmission constructed in accordance with embodiments of the present invention. This test machine is exemplary only, as the torque transfer transmission may be employed in many other applications. However, this depiction of the test machine illustrates some of the features of embodiments of the present invention.

The test machine 30, which in this embodiment can be used for testing orthopedic devices, for example, has a plurality of test stations 32. In the illustrated embodiment, the test machine 30 is a spinal implant wear test machine, although it may be adapted for use in testing other orthopedic and prosthetic devices, or other types of devices. In the illustrated embodiment, there are six test stations 32 in which specimens are subjected to the forces applied by the machine 30, and a control station 34 that holds a specimen that is not subjected to all of the forces provided at the other test stations 32.

In the following description, the test machine 30 will be described as a spinal implant wear test machine for descriptive purposes, although it is to be clearly understood that this machine and the torque transfer or transmission may be employed in other applications. The test machine 30 is able to provide forces Fx, Fy and Fz in the x, y and z directions as depicted in Figure 2, shown with the x, y and z axes at one of the test stations 32. Additionally, torques may be applied around the x, y and z axes, as depicted. The test specimen is not shown in Figure 2 so as not to obscure the present invention. In a spinal implant wear testing machine, a specimen containment module 36 contains fluid in which the spinal implant test specimen is immersed. Upper and lower adapters 38 (only seen clearly at one of the test stations 36 in which the test chamber is removed for illustrative purposes) hold the test specimens between them within the containment module 36.

Briefly, the linkage 40 provides forces in the x direction with the linkage 42 providing forces in the y direction. The gimbals connected to the upper adapters 38 may be moved around the y axis and around the x axis to provide moments around the x and y axes.

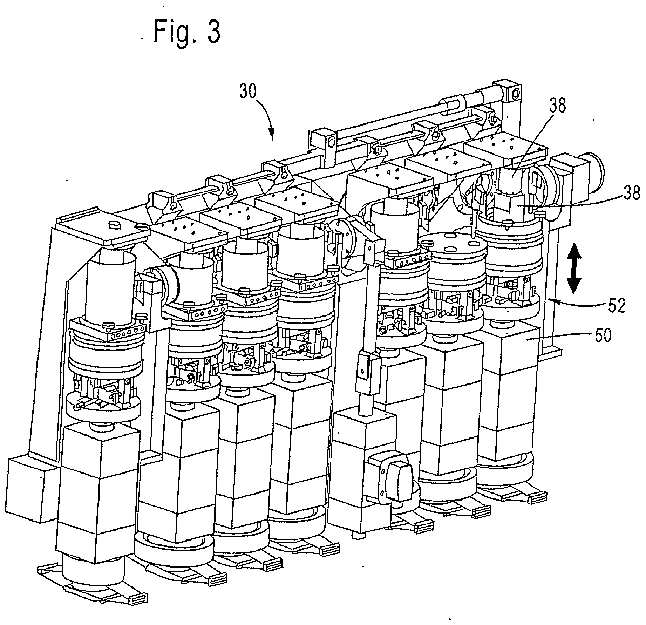

The vertical loads, represented by forces along the z axis, are provided by vertical load actuators 50, as shown in Figure 3. Although different types of actuators may be employed, a friction free axial actuator is preferable to provide for a friction free axial/torsion actuation system. The vertical load actuator 50 applies a vertical loading along the z axis through components 52 to the test specimen (not shown) via the lower adapter 38. In the illustrated embodiment, the components 52 include an x-y slide table and a load cell.

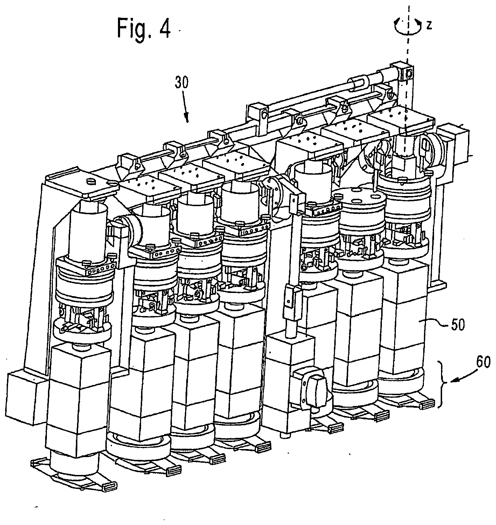

As described earlier, it is desirable to provide a transmission of drive torque with little deflection related error, having high torsional stiffness. At the same time, a low axial stiffness is desirable so that there is little cross-talk onto the vertical loading and so that cross-talk is not seen at the load cell. Figure 4 highlights the axial rotation linkage 60 which is coupled to the actuator 50. The motion of the axial rotation linkage 60 is around the vertical axis z, as depicted in Figure 4. Although the axial rotation linkage 60 is depicted at the bottom of Figure 4, it should be apparent to those of skill in the art that the structure depicted in Figure 4 is suspended vertically so that the axial rotation linkages 60 are free to rotate.

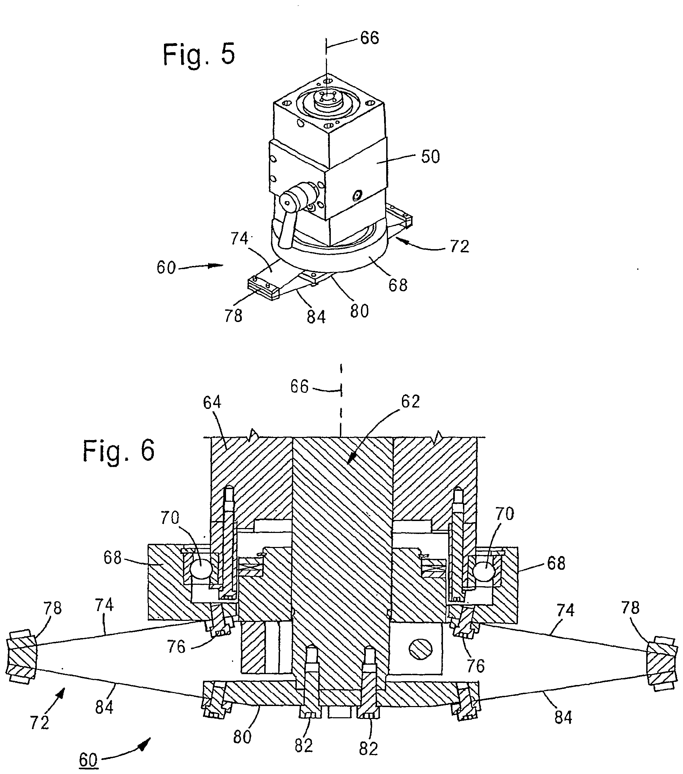

An isolated view of an embodiment of an axial force actuator 50 with axial rotation linkage 60 is depicted in Figure 5. A cross-sectional view of the torque transmitter (or transmission for torque transfer) is depicted in Figure 6. Both figures will be referred to in the following.

The torque transfer transmission 60 includes a rotational element 62, such as an actuator rod 62, that is configured for rotation and axial movement with respect to a stationary (non-rotational) element. In this case, the actuator housing 64 serves as the non-rotational element. The actuator rod 62 has a central axis of rotation 66.

The axial force applicator 50 in preferred embodiments is a friction free axial actuator. Such a friction free axial actuator, combined with the torque transfer transmission of the present invention, provides a friction free axial/torsion actuation system. The actuator rod 62 is allowed to translate along axis 66 freely due to the friction free nature of the axial force applicator, as well as the axial compliance of the torque transfer transmission in accordance with embodiments of the invention. Examples of friction free axial force applicators are known to those of skill in the art. Although not perfectly frictionless, in such an actuator the friction is extremely low and is due only to viscous oil shear.

It is desirable to provide for a torque transfer to the actuator rod 62 with high torsional stiffness while not inducing frictional forces to the actuator rod 62. The torque transfer transmission 60 achieves this by providing a flexible connector 72 that is torsio[pi]ally stiff and axially compliant. A rotational transfer link 68 is rotatably mounted at the bottom of the actuator 50. In the embodiment depicted in Figures 5 and 6, the rotational transfer link 68 is annularly shaped and rotates around the central axis 66. The rotational transfer link 68 is mounted on bearings 70 that allow free rotation of the rotational transfer link 68.

The torque transfer transmission 60 includes the flexible connector 72 (or "axially compliant element") that is coupled between the rotational transfer link 68 and the rotational element (actuator rod) 62. The flexible connector 72 is axially compliant so that it is flexible along the axis of rotation. At the same time, the flexible connector 72 is torsionally stiff so as to rigidly transfer torque between the rotational transfer link 68 and the rotational element (actuator rod) 62.

In embodiments of the invention, such as those depicted in Figures 5 and 6, the flexible connector 72 includes a plurality of flexible elements, such as flexure plates. A first flexure plate 74 is attached to the rotational transfer link 68, such as by fastener 76. The first flexure plate 74 extends at an angle away from the rotational transfer link from a first end to connect at a second end to a connector 78.

A shaft coupler 80 is coupled to the actuator rod 62 by fasteners 82, for example. Second flexure plates 84 are coupled to the shaft coupler 80 at one end, and extend outwardly at an angle from the shaft coupler to the connector 78. The first and second flexure plates are thereby connected at their second ends to the connector 78.

As seen in Figures 5 and 6, two pairs of first and second flexure plates 74, 84 may be arranged 180° from one another. Additional pairs of flexure plates may also be provided in the same plane.

In other embodiments, additional flexible connectors 72 may be provided in series so as to increase axial compliance while maintaining high torsional stiffness.

The embodiments of the present invention that are illustrated depict flexure plates as the flexible connector 72. However, other types of flexible connectors 72 may be employed without departing from the scope of the present invention. For example, flexible billows may be used or other arrangements that provide a flexible connection that is torsionally stiff while being axially compliant.

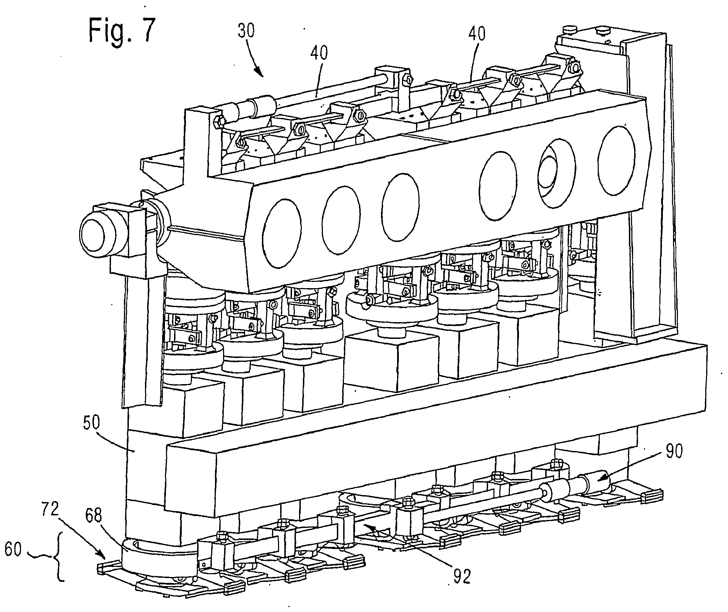

A rear view of the test machine 30 is depicted in Figure 7. A linear actuator 90 operates via a connecting link 92 to drive the plurality of rotational transfer links 68 simultaneously. The linear motion imparted to the connecting link 92 is translated to a rotational motion at the connection to the rotational transfer links 68 at the test stations 32. Hence, all of the rotational transfer links 68 are precisely and simultaneously driven. The axial compliant nature of the torque transfer transmission assures that friction forces do not interfere with providing the same torque to each of the test specimens.

In operation of the test machine 30, the forces in the x and y directions and moments around the x and y axes may be applied and controlled independently from the vertical force application (shown in Figure 3), as well as the axial rotation (shown in Figure 4). A friction free axial/torsion actuation is provided by the illustrated arrangement. The axial force applicator 50 applies a load to the test specimen along the z-axis by moving the actuator rod 62 along the z-axis. Driven by linear actuator 90 through the connecting link 92, the rotational transfer link 68 and the flexible connector 72 facilitate rigid torque transfer to the actuator rod 62 to the test specimen (not shown) at the test station 32. The actuator rod 62 is allowed to translate along its axis 66 freely due to the high axial compliance provided by the flexible connector 72 of the torque transfer transmission 60. The arrangement may be used with precise force and motion control applications, such as in test machines for testing wear of orthopedic and prosthetic devices. The arrangement may also be used in other devices in which a torque transfer is desired without inducing frictional forces.

Although embodiments of the present invention have been described and illustrated in detail, it is to be clearly understood that the same is by way of illustration and example only and is not to be taken by way of limitation, the scope of the present invention being limited only by the terms of the appended claims.

## Claims

1. A transmission (10, 60) for torque transfer, comprising:
a rotational element (12, 62) configured to supply a load to a test specimen, the rotational element (12, 62) configured for rotation about an axis of rotation (20, 66) relative to a non-rotational element (64) and axially movable along the axis of rotation (20, 66) with respect to the non-rotational element (64);
a rotational transfer link (68) drivable by an actuator (50); and
a flexible connector (72) which is flexible along the axis of rotation and coupled between the rotational transfer link (68) and the rotational element (12, 62), **characterized in that**
the rotational transfer link (68) is rotatable about the axis of rotation (20, 66); and
the flexible connector (72) includes flexible elements (74, 84) which are torsionally stiff to rigidly transfer torque between the rotational transfer link (68) and the rotational element (12, 62); wherein the flexible connector (72) includes a first flexure plate (74) attached at a first end to the rotational transfer link (68) and extending outwardly from the rotational transfer link (68) and having a second end, and a second flexure plate (84) attached at a first end to a shaft coupler (80) connected to the rotational element (12, 62) and extending outwardly from the shaft coupler (80) and having a second end coupled to the second end of the first flexure plate (74) through a connector (78).

2. The transmission of claim 1, wherein the rotational element is an actuator rod (62) and the non-rotational element (64) is a linear actuator housing through which the actuator rod (62) extends.

3. The transmission of claim 2, wherein the rotational transfer link (68) is annular and is rotationally coupled by bearings (70) to the linear actuator housing (64).

4. The transmission of claim 1 wherein the first end of the first flexure plate (74) is connected to the rotational transfer link (68) and extends at an angle away from the rotational transfer link (68) to connect at the second end to the connector (78), and wherein the first end of the second flexure plate (84) is connected to the rotational element (12, 62) and extends at an angle away from the rotational element (12, 62) to connect at the second end to the connector (78).

5. The transmission of claim 4, wherein the rotational element (12, 62) includes the shaft coupler (80) connected to an end of a rotary shaft and having connection surfaces at which the second flexure plate (84) is connected.

6. The transmission of claim 5, wherein the flexible connector (72) includes a pair of first flexible elements (74) and a pair of second flexible elements (84), with one set of the first and second flexible elements being arranged 180° from another set of the first and second flexible elements.

7. The transmission of claim 1, further comprising a plurality of flexible connectors coupled in series.

8. An orthopedic device test machine (30), comprising:
at least one test station (32) comprising the transmission of claim 1.

9. The orthopedic device test machine of claim 8, wherein the rotational element (12, 62) is an axially translatable, rotatable shaft (60) in an axial actuator (50).

10. The orthopedic device test machine of claim 9, wherein the axial actuator (50) is a friction free axial actuator.

11. The orthopedic device test machine of claim 10, wherein the second flexure plate (84) is coupled at the first end to the axial actuator (50) and extends outwardly from the axial actuator (50).

12. The orthopedic device test machine of claim 8, further comprising a plurality of axially compliant elements coupled in series.

## Patentansprüche

1. Übersetzung (10, 60) zur Drehmomentübertragung, mit:
einem Drehelement (12, 62), das dafür konfiguriert ist, einem Probekörper eine Last zuzuführen, wobei das Drehelement (12, 62) für eine Drehbewegung um eine Drehachse (20, 66) relativ zu einem nicht drehbaren Element (64) konfiguriert und entlang der Drehachse (20, 66) bezüglich des nicht drehbaren Elements (64) axial beweglich ist;
einem durch einen Aktuator (50) antreibbaren
Drehbewegungsübertragungsverbindungsstück (68); und
einem flexiblen Verbinder (72), der entlang der Drehachse flexibel ist und zwischen das Drehbewegungsübertragungsverbindungsstück (68) und das Drehelement (12, 62) gekoppelt ist;
**dadurch gekennzeichnet, dass**
das Drehbewegungsübertragungsverbindungsstück (68) um die Drehachse (20, 66) drehbar ist; und
der flexible Verbinder (72) flexible Elemente (74, 84) aufweist, die verwindungssteif sind, um ein Drehmoment zwischen dem Drehbewegungsübertragungsverbindungsstück (68) und dem Drehelement (12, 62) starr zu übertragen, wobei der flexible Verbinder (72) aufweist:
eine erste Biegeplatte (74), die an einem ersten Ende mit dem Drehbewegungsübertragungsverbindungsstück (68) verbunden ist und sich vom Drehbewegungsübertragungsverbindungsstück (68) nach außen erstreckt und ein zweites Ende aufweist; und
eine zweite Biegeplatte (84), die an einem ersten Ende an einem Wellenverbinder (80) befestigt ist, der mit dem Drehelement (12, 62) verbunden ist, und der sich vom Wellenverbinder (80) nach außen erstreckt und ein zweites Ende aufweist, das über einen Verbinder (78) mit dem zweiten Ende der ersten Biegeplatte (74) verbunden ist.

2. Übersetzung nach Anspruch 1, wobei das Drehelement eine Aktuatorstange (62) ist und das nicht drehbare Element (64) ein Gehäuse für einen linearen Aktuator ist, durch das sich die Aktuatorstange (62) erstreckt.

3. Übersetzung nach Anspruch 2, wobei das
Drehbewegungsübertragungsverbindungsstück (68) ringförmig ausgebildet ist und durch Lager (70) mit dem Gehäuse (64) für einen linearen Aktuator drehbar verbunden ist.

4. Übersetzung nach Anspruch 1, wobei das erste Ende der ersten Biegeplatte (74) mit dem Drehbewegungsübertragungsverbindungsstück (68) verbunden ist und sich in einem Winkel vom Drehbewegungsübertragungsverbindungsstück (68) weg erstreckt, so dass sie am zweiten Ende mit dem Verbinder (78) verbunden ist, und wobei das erste Ende der zweiten Biegeplatte (84) mit dem Drehelement (12, 62) verbunden ist und sich in einem Winkel vom Drehelement (12, 62) weg erstreckt, so dass sie am zweiten Ende mit dem Verbinder (78) verbunden ist.

5. Übersetzung nach Anspruch 4, wobei das Drehelement (12, 62) den Wellenverbinder (80) aufweist, der mit einem Ende einer Drehwelle verbunden ist und Verbindungsflächen aufweist, mit denen die zweite Biegeplatte (84) verbunden ist.

6. Übersetzung nach Anspruch 5, wobei der flexible Verbinder (72) ein Paar erste flexible Elemente (74) und ein Paar zweite flexible Elemente (84) aufweist, wobei ein Satz der ersten und zweiten flexiblen Elemente um 180° von einem anderen Satz der ersten und zweiten flexiblen Elemente versetzt angeordnet ist.

7. Übersetzung nach Anspruch 1, ferner mit mehreren in Serie verbundenen flexiblen Verbindern.

8. Prüfmaschine (30) für eine orthopädische Vorrichtung, mit:
mindestens einer Prüfstation (32) mit der Übersetzung nach Anspruch 1.

9. Prüfmaschine nach Anspruch 8, wobei das Drehelement (12, 62) eine axial verschiebbare, drehbare Welle (60) in einem axialen Aktuator (50) ist.

10. Prüfmaschine nach Anspruch 9, wobei der axiale Aktuator (50) ein reibungsfreier axialer Aktuator ist.

11. Prüfmaschine nach Anspruch 10, wobei die zweite Biegeplatte (84) am ersten Ende mit dem axialen Aktuator (50) verbunden ist und sich vom axialen Aktuator (50) nach außen erstreckt.

12. Prüfmaschine nach Anspruch 8, ferner mit mehreren in Serie verbundenen axial nachgiebigen Elementen.

## Revendications

1. Transmission (10, 60) pour transfert de couple, comprenant :
un élément rotatif (12, 62) configuré pour fournir une charge à un spécimen d'essai, l'élément rotatif (12, 62) étant configuré pour tourner autour d'un axe de rotation (20, 66) par rapport à un élément non-rotatif (64) et étant axialement mobile le long de l'axe de rotation (20, 66) par rapport à l'élément non rotatif (64) ;
une liaison de transfert rotative (68) qui peut être entraînée par un actionneur (50) ; et
un raccord flexible (72) qui est flexible le long de l'axe de rotation et relié entre la liaison de transfert rotative (68) et l'élément rotatif (12, 62),
**caractérisé en ce que**
la liaison de transfert rotative (68) peut tourner autour de l'axe de rotation (20, 66) ; et
le raccord flexible (72) comprend des éléments flexibles (74, 84) qui sont rigides en torsion afin de transférer le couple de manière rigide entre la liaison de transfert rotative (68) et l'élément rotatif (12, 62) ;
dans laquelle le raccord flexible (72) comprend une première plaque de flexion (74) fixée au niveau d'une première extrémité sur la liaison de transfert rotative (68) et s'étendant vers l'extérieur depuis la liaison de transfert rotative (68) et ayant une seconde extrémité, et une seconde plaque de flexion (84) fixée au niveau d'une première extrémité sur un coupleur d'arbre (80) relié à l'élément rotatif (12, 62) et s'étendant vers l'extérieur depuis le coupleur d'arbre (80) et ayant une seconde extrémité reliée à la seconde extrémité de la première plaque de flexion (74) par le biais d'un raccord (78).

2. Transmission selon la revendication 1, dans laquelle l'élément rotatif est une tige d'actionnement (62) et l'élément non-rotatif (64) est un boîtier d'actionneur linéaire dans lequel s'étend la tige d'actionnement (62).

3. Transmission selon la revendication 2, dans laquelle la liaison de transfert rotative (68) est annulaire et est reliée de manière rotative par des roulements (70) au boîtier d'actionneur linéaire (64).

4. Transmission selon la revendication 1 dans laquelle la première extrémité de la première plaque de flexion (74) est reliée à la liaison de transfert rotative (68) et s'étend à un certain angle à l'écart de la liaison de transfert rotative (68) de façon à être reliée, au niveau de la seconde extrémité, au raccord (78), et dans laquelle la première extrémité de la seconde plaque de flexion (84) est reliée à l'élément rotatif (12, 62) et s'étend à un certain angle à l'écart de l'élément rotatif (12, 62) de façon à être reliée, au niveau de la seconde extrémité, au raccord (78).

5. Transmission selon la revendication 4, dans laquelle l'élément rotatif (12, 62) comprend le coupleur d'arbre (80) relié à une extrémité d'un arbre rotatif et ayant des surfaces de liaison au niveau desquelles la seconde plaque de flexion (84) est reliée.

6. Transmission selon la revendication 5, dans laquelle le raccord flexible (72) comprend une paire de premiers éléments flexibles (74) et une paire de seconds éléments flexibles (84), un ensemble des premier et des seconds éléments flexibles étant disposé à 180° d'un autre ensemble des premier et des seconds éléments flexibles.

7. Transmission selon la revendication 1, comprenant en outre une pluralité de raccords flexibles reliés en série.

8. Machine d'essai de dispositif orthopédique (30), comprenant :
au moins un poste d'essai (32) comprenant la transmission selon la revendication 1.

9. Machine d'essai de dispositif orthopédique selon la revendication 8, dans laquelle l'élément rotatif (12, 62) est un arbre rotatif à translation axiale (60) dans un actionneur axial (50).

10. Machine d'essai de dispositif orthopédique selon la revendication 9, dans laquelle l'actionneur axial (50) est un actionneur axial sans frottement.

11. Machine d'essai de dispositif orthopédique selon la revendication 10, dans laquelle la seconde plaque de flexion (84) est reliée, au niveau de la première extrémité, à l'actionneur axial (50) et s'étend vers l'extérieur depuis l'actionneur axial (50).

12. Machine d'essai de dispositif orthopédique selon la revendication 8, comprenant en outre une pluralité d'éléments axialement souples reliés en série.
